# EUROPEAN PATENT APPLICATION

(11) **EP 1 329 431 A1**
(43) Date of publication of application: **23.07.2003**
(21) Application number: 02291038.4
(22) Date of filing: 24.04.2002
(51) Int. Cl.: C03C 3/097, C03C 8/02, C03C 4/00, C04B 35/16, H05B 3/00

(54) **Far-infrared ray radiator and method for manufacturing the same**

(30) Priority: 21.01.2002 KR 2002003402
(71) Applicant: Son, Sang-Ho, Sungnam-Si, Kyungki-Do (KR)
(72) Inventor: Son, Sang-Ho, Sungnam-Si, Kyungki-Do (KR)
(74) Representative: Lemoine, Robert

(57) **Abstract**

The present invention relates to a radiator emitting far-infrared rays and anions, and a method for manufacturing the radiator. The radiator according to the present invention is characterized in that it has a high far-infrared ray emissivity, and simultaneously emits a large quantity of anions to be utilized in the human body. To manufacture the far-infrared ray radiator, silicon oxide of 50 to 55 parts by weight, potassium oxide of 2 to 4 parts by weight, alumina of 3 to 7 parts by weight, borax of 15 to 25 parts by weight, sodium oxide of 10 to 20 parts by weight and calcium oxide of 3 to 7 parts by weight are introduced into a vessel and then mixed for 30 to 60 minutes. The mixture is put into a melting furnace at 1200 to 1400 °C and calcinated for 1 to 3 hours. The calcinated material is quenched and then pulverized. The pulverized material was added with phosphate rock of 1 to 5 parts by weight and then pulverized. In this way, the far-infrared ray radiator according to the present invention is obtained.

## Description

### BACKGROUND OF THE INVENTION

### Field of the Invention

The present invention relates to a radiator emitting far-infrared rays and anions and a method for manufacturing the radiator. More particularly, the present invention relates to a radiator having a high far-infrared ray emissivity, and simultaneously being capable of emitting a large quantity of anions to promote the health of the human body, and a method for manufacturing the radiator.

### Description of the Related Art

Generally, far-infrared rays is a general term for infrared rays with a wavelength range of 3 to 1000 µm. The wavelengths of the far-infrared rays are longer than those of visible rays while being shorter than those of microwaves. The far-infrared rays have characteristics of resonance absorption, radiation and deep penetration capability.

Resonance absorption is such a process that when a substance is irradiated with a far-infrared ray having the same frequency as the substance's molecules, the molecules absorb the radiant energy and thus the substance's molecular vibration is increased. Part of kinetic energy is converted into activation energy by resonance absorption, and activation energy enhances molecular motion. Radiation refers to a conversion of far-infrared rays radiated from a substance into heat. Deep penetration capability is determined in proportion to the square root of the frequency of a radiant energy being radiated. Accordingly, far-infrared rays with short wavelengths have lower penetration capabilities compared with far-infrared rays with long wavelengths.

Owing to the above described characteristics, the far-infrared rays deeply penetrate into the human body to activate molecules or atoms of the body. Accordingly, they eliminate various wastes matters of the body and promote metabolism, activating the biorhythm (for example, cell generation, recovery from fatigue and the like).

Meanwhile, materials emitting anions are crystals with electric polarization. In the unit lattice of the crystal, since centers of cations and anions are deviated from their intrinsic positions, at opposite sides of the crystal are formed an anode and a cathode, respectively, and electrons moving toward the anode are generated from the cathode.

Electrons generated from the cathode are combined with other atoms to form anions. The anions formed move along electric field lines and then become permanent currents. The currents are micro currents of about 0.06 mA and most suitable for the activation of physical function.

When the anion emitting material is in contact with water, the material is instantaneously discharged, so that water molecules are electrically split into hydrogen ions(H⁺) and hydroxyl ions(OH⁻). At this time, the hydrogen ions(H⁺) are deoxidized by the electrons emitted from the cathode into hydrogen gas(H²) and then vaporized. Accordingly, water is alkalinized. Also, the hydroxyl ions(OH⁻) serve to deoxidize an acidified body, enhance immune function(sterilizing power and antimicrobial activity) by lightly alkalizing water, purify the blood and inhibit excitation of the sympathetic nerves by stimulating the autonomic nerves.

Because of the above described virtues of the materials emitting the far-infrared rays and materials emitting anions, recently, various studies for utilizing these materials in health products of daily life have been carried out. However, there is a disadvantage in that since conventional far-infrared ray radiators are used without any processing in their raw forms such as clay or raw ore, they have low emitting amounts as well as low emissivities.

Therefore, the present invention proceeded from results of studies on radiators having high far-infrared ray emissivities, and simultaneously being capable of emitting a large quantity of anions to be utilized in the human body.

### SUMMARY OF THE INVENTION

Therefore, the present invention has been made in view of the above problems, and it is an object of the present invention to provide a far-infrared ray radiator having a high far-infrared ray emissivity, and simultaneously being capable of emitting a large quantity of anions to be utilized in the human body, and a method for manufacturing the radiator.

In accordance with the present invention, the above and other objects can be accomplished by the provision of a method for manufacturing a far-infrared ray radiator comprising the steps of:
introducing silicon oxide of 50 to 55 parts by weight, potassium oxide of 2 to 4 parts by weight, alumina of 3 to 7 parts by weight, borax of 15 to 25 parts by weight, sodium oxide of 10 to 20 parts by weight and calcium oxide of 3 to 7 parts by weight into a vessel, and mixing them for 30 to 60 minutes;
putting the mixture into a melting furnace at 1200 to 1400 °C, and calcinating the mixture for 1 to 3 hours;
quenching the calcinated material, and pulverizing the quenched material; and
adding phosphate rock of 1 to 5 parts by weight to the quenched material, and pulverizing the resultant material.

### DESCRIPTION OF THE PREFERRED EMBODIMENTS

The present invention will hereinafter be described in more detail.

In accordance with the present invention, to manufacture a far-infrared ray radiator, silicon oxide of 50 to 55 parts by weight, potassium oxide of 2 to 4 parts by weight, sodium oxide of 10 to 20 parts by weight and calcium oxide of 3 to 7 parts by weight are introduced in a vessel and then mixed for 30 to 60 minutes.

Since silicon oxide has an excellent emissivity, it has been generally used in the manufacture of radiators. In the present invention, silicon oxide was used in an amount of 50 to 55 parts by weight, which is within the range of normal use.

To manufacture a far-infrared ray radiator with a high emissivity, it is required to calcinate silicon oxide at a high temperature. However, where silicon oxide is calcinated at excessively high temperature, it causes damage to equipment due to corrosion of a melting furnace, and impurities may be introduced, so that there is a disadvantage in that the emissivity of the far-infrared rays is reduced.

To solve the problem, the melting temperature needs to be decreased. In the present invention, part of silicon oxide was substituted with borax and alumina to decrease the melting temperature.

The added amounts of borax and alumina are respectively determined based on their dissolved amounts into silicon oxide. In the present invention, alumina of 3 to 7 parts by weight and borax of 15 to 25 parts by weight were used.

Here, alumina acts to loosen the structure of silicon oxide through the substitution reaction therewith at a high temperature to decrease the melting temperature. Where the added amount of alumina is less than 3 parts by weight, it is difficult to sufficiently obtain the effect of decreasing the melting temperature. Meanwhile, where the added amount of alumina is more than 7 parts by weight, the substitution limit of alumina for silicon oxide is exceeded, resulting in an increase of the melting temperature. Accordingly, it is preferable that alumina is added in an amount of 3 to 7 parts by weight.

Also, borax acts to loosen the structure of silicon oxide through the substitution reaction therewith at a high temperature to decrease the melting temperature. Where the added amount of borax is less than 15 parts by weight, it is difficult to sufficiently obtain the effect of decreasing the melting temperature. Meanwhile, where the added amount of borax is more than 25 parts by weight, the structure of silicon oxide is loosened, resulting in a poor radiator. Accordingly, it is preferable that borax is added in an amount of 15 to 25 parts by weight.

In accordance with the present invention, potassium oxide and calcium oxide are also added to increase the far-infrared ray emissivity while simultaneously decreasing the calcination temperature. The added potassium oxide and calcium oxide act to cut bonds between atoms in silicone oxide. Accordingly, the calcination temperature may be decreased and simultaneously the atomic structure of a far-infrared ray radiator manufactured becomes irregular, thereby causing the atomic bonds to be loosed on the whole. Owing to such a change, the radiator emits a large quantity of far-infrared rays in a region of short wavelengths, which are beneficial to the human body.

Where the added amount of potassium oxide is less than 2 parts by weight, it is difficult to sufficiently obtain the effect of decreasing the melting temperature. Meanwhile, where the added amount of potassium oxide is more than 4 parts by weight, potassium oxide is eluted, thereby causing a whitening phenomenon. Accordingly, it is preferable that potassium oxide is added in an amount of 2 to 4 parts by weight.

Where the added amount of sodium oxide is less than 10 parts by weight, it is difficult to sufficiently obtain the effect of decreasing the melting temperature. Meanwhile, where the added amount of sodium oxide is more than 20 parts by weight, there occurs a problem that potassium oxide is eluted. Accordingly, it is preferable that sodium oxide is added in an amount of 10 to 20 parts by weight.

As described above, when potassium oxide and calcium oxide are added, by contact with water, some of the material is ionized and then eluted, leading to a whitening phenomenon. Accordingly, in the present invention, calcium oxide was added in an amount of 3 to 7 parts by weight to solve this problem.

Calcium ions are mixed with alkali ions such as potassium and sodium ions by addition of calcium oxide. This prevents the elution of potassium and sodium ions, and at the same time increases the toughness of materials.

Silicon oxide, potassium oxide, alumina, borax, sodium oxide and calcium oxide, which are within the ranges described above, are introduced in a vessel and mixed for 30 to 60 minutes. Then, the mixture is put into a melting furnace and subjected to calcination. The molten material is quenched and then pulverized.

At this time, where the calcination temperature is less than 1200 °C, there is a disadvantage in that the additives are not sufficiently melted, thereby making it difficult to obtain a radiator with a homogeneous characteristic. Where the calcination temperature is more than 1400 °C, it causes the mixing of impurities caused by corrosion of a melting furnace, so that there is a disadvantage in that the far-infrared ray emissivity is reduced. Accordingly, it is preferable that the calcination temperature is within the range described above.

After the calcination, where the molten material is slowly cooled, it is crystallized, leading to reduction of radiation efficiency. For this reason, in the present invention, the molten material was quenched. At this time, the quenching was carried out using water of room temperature.

The quenched material is pulverized to an appropriate grain size according to the application of a radiator to be manufactured.

Now, a radiator is manufactured by adding phosphate rock to the pulverized material thus prepared in an amount of 1 to 5 parts by weight. At this time, phosphate rock is added to obtain the emitting effect of anions. Where the added amount of phosphate rock is less than 1 part by weight, it is difficult to sufficiently obtain the effect of emission of anions. Where the added amount is more than 5 parts by weight, there is a disadvantage in that the radiation amount of the far-infrared ray radiator is reduced. Accordingly, it is preferable that phosphate rock is added within the range described above.

Phosphate rock may be pulverized, prior to its addition into the pulverized material, to the same grain size as the material. Alternatively, phosphate rock may be pulverized, after its addition, considering the radiator's application.

The far-infrared ray radiator thus manufactured according to the present invention has a maximum wavelength in the range of 5 to 20 µm. Also, since the radiator has a very high far-infrared ray emissivity of about 0.94, its far-infrared ray radiation effect is maximized. Additionally, the radiator emits a large amount of anions. Therefore, the radiator according to the present invention can be usefully utilized for promoting the health of the human body.

Particularly, since the far-infrared ray radiator according to the present invention has a high far-infrared ray emissivity and simultaneously emits a large quantity of anions, it can be utilized for various uses for health promotion. The far-infrared ray radiator thus manufactured may properly be subjected to post-process phases according to its uses.

Now, examples of the present invention will be described in more detail. The following examples are described only to provide a better understanding of the present invention, and are not intended to limit the invention.

### <Example 1>

1040g of silicon oxide, 60g of potassium oxide, 100g of alumina, 400g of borax, 300g of sodium oxide and 100g of calcium oxide were introduced in a vessel and mixed for 30 minutes. The mixture was putted to a melting furnace at 1300 °C, and then calcinated for 2 hours. The calcinated material was quenched and then pulverized into powder of 200 mesh. The pulverized material was added with 100g of phosphate rock and then pulverized into powder of 200 mesh. In this way, a far-infrared ray radiator was obtained in the form of powder.

The far-infrared ray radiator powder was heated at 30 °C. Then, the emissivity and radiant energy were measured by FT-IR(US MIDAC co.; Model No: M 2400-C) using the heated radiator powder. Also, the concentration of anions was measured by a ultra-high sensitive microcurrent detector(Japan Shinho electronics co.; Model No: KST900) using the heated radiator powder. Measurements of the emissivity and radiant energy, and the concentration of anions are described in the following Table 1.

### <Example 2>

1100g of silicon oxide, 60g of potassium oxide, 80g of alumina, 360g of borax, 300g of sodium oxide and 100g of calcium oxide were introduced into a vessel and mixed for 30 minutes. The mixture was put into a melting furnace at 1300 °C and calcinated for 2 hours. The calcinated material was quenched and then pulverized into powder of 200 mesh. The pulverized material was added with 100g of phosphate rock and then pulverized into powder of 200 mesh. In this way, a far-infrared ray radiator was obtained in the form of powder.

The emissivity and radiant energy, and the concentration of anions were measured in the same manner as in Example 1 using the far-infrared ray radiator powder. The results are described in the following Table 1.

### <Comparative Example 1>

2kg of clay composed of 53.56% silicon oxide, 30.67% alumina, 1.16% ferric oxide, 0.20% calcium oxide, 0.22% magnesium oxide, 0.95% titanium oxide, 1.07% potassium oxide and 12.17% other combustion losses was putt into a melting furnace at 1400 C and calcinated for 2 hours. The calcinated clay was quenched and then pulverized into powder of 200 mesh. In this way, a far-infrared ray radiator was obtained.

The emissivity and radiant energy, and the concentration of anions were measured in the same manner as in Example 1 using the far-infrared ray radiator powder. The results are described in the following Table 1.

**Table 1**

| Examples | Far-infrared ray emissivity | Radiant energy(W/m²· µm) | Concentration of anions(No./mℓ) |
|---|---|---|---|
| Ex. 1 | 0.94 | 4.4 × 10² | 1500 |
| Ex. 2 | 0.93 | 4.4 × 10² | 1500 |
| Com. Ex. 1 | 0.89 | 3.9 × 10² | 30 |

As shown in Table 1, it was found that the far-infrared ray radiators of Examples 1 and 2 according to the present invention have very high far-infrared ray emissivities and emit a large quantity of anions, compared with the conventional far-infrared ray radiator.

Therefore, the far-infrared ray radiator according to the present invention can obtain both the benefits of by far-infrared ray radiation and benefits of generation of anions, so that it can be usefully utilized for promoting the health of the human body.

As apparent from the above description, the present invention provides a far-infrared ray radiator, which can radiate far-infrared rays at a high emissivity and simultaneously emit a large quantity of anions, so that it can be usefully utilized for promoting the health of the human body, and a method for manufacturing the radiator.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A method for manufacturing a far-infrared ray radiator comprising the steps of:
introducing silicon oxide of 50 to 55 parts by weight, potassium oxide of 2 to 4 parts by weight, alumina of 3 to 7 parts by weight, borax of 15 to 25 parts by weight, sodium oxide of 10 to 20 parts by weight and calcium oxide of 3 to 7 parts by weight into a vessel, and mixing them for 30 to 60 minutes;
putting the mixture into a melting furnace at 1200 to 1400 °C, and calcinating the mixture for 1 to 3 hours;
quenching the calcinated material, and pulverizing the quenched material; and
adding phosphate rock of 1 to 5 parts by weight to the quenched material, and pulverizing the resultant material.

2. A far-infrared ray radiator manufactured by a method according to claim 1.
